# EUROPÄISCHE PATENTANMELDUNG

(11) **EP 3 670 105 A1**
(43) Veröffentlichungstag der Anmeldung: **24.06.2020**
(21) Anmeldenummer: 18212922.1
(22) Anmeldetag: 17.12.2018
(51) Int. Cl.: B25J 9/00, A61F 5/01, A61H 1/02, A61F 2/78

(54) **ÜBERKOPF-EXOSKELETT**

(71) Anmelder: Hilti Aktiengesellschaft, 9494 Schaan (LI)
(72) Erfinder: Käsmann, Simon, 86916 Kaufering (DE); Melzer, Lars, 86978 Hohenfurch (DE)
(74) Vertreter: Hilti Aktiengesellschaft Corporate Intellectual Property

(57) **Zusammenfassung**

Exoskelett (10) zum Stützen wenigstens eines Arms (20) eines Anwenders (AW) mit einer Rumpfanbindungsvorrichtung (30) zum wiederlösbaren Verbinden des Exoskeletts (10) mit dem Rumpf des Anwenders (AW), einer Halterung (9) zum Verbinden mit der Rumpfanbindungsvorrichtung (30), einer Hubstange (7), welche reversibel in eine erste und zweite Richtung (A, B) relativ zu der Halterung (9) bewegbar ist, einem Ausleger (4) zur wiederlösbaren Verbindung mit dem Arm (20) des Anwenders (AW); und einem Federelement (60) zum Ausüben einer Kraft auf den Ausleger (4) und die Hubstange (7).

## Beschreibung

Die vorliegende Erfindung betrifft ein Exoskelett zum Stützen wenigstens eines Arms eines Anwenders.

Auf dem Markt erhältliche Exoskelette, die zur passiven und/oder aktiven Unterstützung der Extremitäten eines Anwenders, insbesondere der Arme, verfügbar sind, enthalten für gewöhnlich eine Hüftbefestigung, eine erste und zweite Stützstrebe sowie eine erste und zweite Armstütze. An der linken und rechten Seite der Hüftbefestigung ist jeweils die erste und zweite Stützstrebe vertikal befestigt. An dem oberen Ende der ersten Stützstrebe ist die erste Armstütze über ein Drehlager drehbar befestigt. Ebenso ist an dem oberen Ende der zweiten Stützstrebe die zweite Armstrebe über ein weiteres Drehlager drehbar befestigt. Die Armstützen lassen sich in unterschiedlichen Winkelpositionen relativ zur Stützstrebe fixieren, sodass die Arme des Anwenders auf den aufgestellten Armstützen abgelegt werden können. Mit Hilfe von Federn können die Armstützen die Arme des Anwenders nach oben drücken und dadurch das Anheben einer großen Last (d.h. mit einem großen Gewicht) erleichtern.

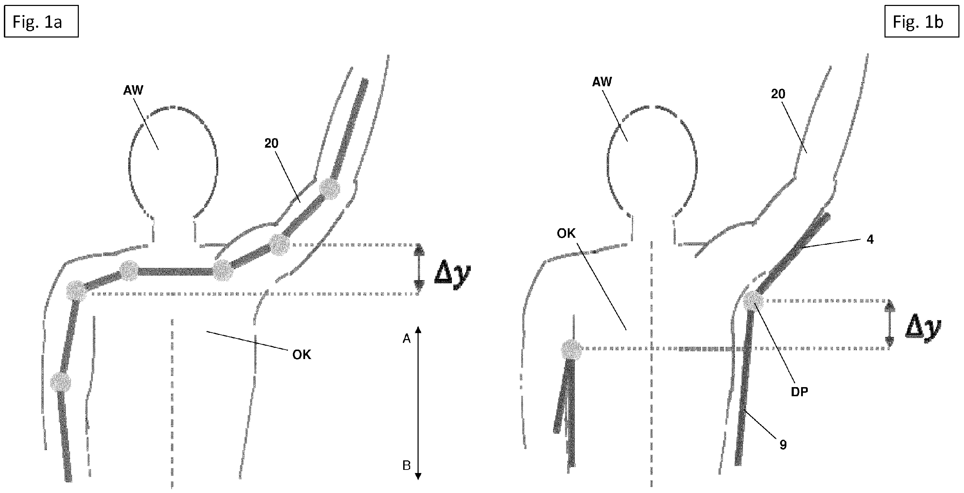

Aus der Anatomie der menschlichen Schulter ergibt sich, wie in Figur 1a schematisch gezeigt, dass bei einem Anheben der Arme gleichzeitig eine Elevation des Schultergelenks stattfindet. Bei Exoskeletten gemäß dem Stand der Technik verbleiben die Drehlager zwischen einer oberen Stützstrebe und Armstütze jedoch an einer Position und folgen nicht der Elevation des menschlichen Schultergelenks. Hierdurch kann es zu einer störenden Relativbewegung zwischen dem Exoskelett und den Armen kommen. Dies führt entweder dazu, dass die Arme auf eine vom Nutzer nicht gewollte Bewegungsbahn gezwungen wird oder die Anbindungspunkte zwischen Exoskelett und menschlichem Körper verrutschen. Daraus resultieren Bewegungseinschränkungen und ein geringer Tragekomfort. Ein derartiges Exoskelett gemäß dem Stand der Technik ist beispielsweise in den Patentschriften US 9,205,017 BB und US 9,737, 374 BB gezeigt.

Es ist die Aufgabe der vorliegenden Erfindung, das vorstehend genannte Problem zu lösen und insbesondere ein verbessertes Exoskelett zum Stützen wenigstens eines Arms eines Anwenders bereitzustellen.

Diese Aufgabe wird gelöst durch ein Exoskelett zum Stützen wenigstens eines Arms eines Anwenders.

Erfindungsgemäß enthält das Exoskelett eine Rumpfanbindungsvorrichtung zum wiederlösbaren Verbinden des Exoskeletts mit einem Rumpf eines Anwenders, wenigstens eine Halterung, welche mit der Rumpfanbindungsvorrichtung verbindbar ist, eine Hubstange, welche mittels der wenigstens eine Halterung mit der Rumpfanbindungsvorrichtung verbindbar ist und welche reversibel in eine erste und zweite Richtung relativ zu der Halterung bewegbar ist, einen Ausleger zum Stützen des Arms eines Anwenders, welcher mit dem Arm eines Anwenders wiederlösbar verbindbar ist und ein Federelement zum Ausüben einer Kraft auf den Ausleger und die Hubstange. Hierdurch wird ein Exoskelett zur Verfügung gestellt, welches eine Ablagemöglichkeit für die Arme eines Anwenders ermöglicht, die an die Elevation des menschlichen Schultergelenks beim Heben des Armes angepasst ist. Durch diese ergonomisch bzw. anatomisch korrekte Ablagemöglichkeit für die Arme eines Anwenders kann eine bessere Unterstützung beim Halten von schweren Lasten für den Anwender erreicht werden.

Entsprechend einer vorteilhaften Ausführungsform der vorliegenden Erfindung kann eine Koppelstange mit einem ersten und zweiten Ende, welche mit dem ersten Ende an dem Ausleger und dem zweiten Ende über einen Ausgleichsmechanismus mit der Hubstange jeweils drehbar verbunden ist. Durch die Koppelstange die Bewegung des Auslegers relativ zu der Hubstange geführt und gestützt werden. Eine ungewollte Seitenbewegung des Auslegers bzw. ein Verdrehen oder eine Torsion des Auslegers gegenüber der Hubstange und/oder Halterung kann hierdurch verhindert werden.

Gemäß einer vorteilhaften Ausführungsform der vorliegenden Erfindung kann der Ausgleichsmechanismus in Form eines Langlochs am zweiten Ende der Koppelstange und eines Drehlagers an der Hubstange ausgestaltet sein, wobei das Drehlager in dem Langloch positioniert ist. Hierdurch kann der Ausgleichsmechanismus auf einfache Art und Weise umgesetzt werden. Darüber hinaus kann durch das Langloch am Ende der Koppelstange der Ausleger gesenkt (d.h. nach unten bewegt) werden, ohne eine Kraft auf weitere Komponenten auszuüben.

Entsprechend einer vorteilhaften Ausführungsform der vorliegenden Erfindung kann der Ausgleichsmechanismus in Form Kulissensteuerung zwischen dem zweiten Ende der Koppelstange und der Hubstange ausgestaltet ist. Hierdurch ist eine sichere Führung des zweiten Endes der Koppelstange gewährleistet.

Entsprechend einer vorteilhaften Ausführungsform der vorliegenden Erfindung kann der Ausgleichsmechanismus in Form in eines Sektorgetriebes sowie einer Zahnstange ausgestaltet ist, wobei das Sektorgetriebe drehbar mit der Hubstange verbunden sein, sodass die Zähne des Sektorgetriebes in die Zähne der Zahnstange eingreifen. Das Sektorgetriebe kann auch als Teilzahnrad bezeichnet werden. Hierdurch kann auf einfache Art und Weise eine effektive Führung zum Vermeiden einer ungewollten Seitenbewegung des Auslegers bzw. ein Verdrehen oder eine Torsion des Auslegers gegenüber der Hubstange und/oder Halterung erreicht werden. Mit Hilfe des Langlochs am Ende der Koppelstange kann der Ausleger gesenkt (d.h. nach unten bewegt) werden, ohne eine Kraft auf das Sektorgetriebe auszuüben.

Entsprechend einer vorteilhaften Ausführungsform der vorliegenden Erfindung kann anstelle des Ausgleichsmechanismus die Gelenkverbindung zwischen dem Ausleger und der Hubstange durch eine Spindel oder einen Aktor ausgeführt sein, um den Ausleger nach oben zu bewegen. Anstelle einer mechanischen Kopplung zwischen dem Ausleger und der Hubstange kann die Winkellage bzw. der Winkel der Armstrebe zu der Hubstange auch über eine Sensorik bestimmt werden, um die Elevation des Auslegers korrekt zu bestimmen.

Gemäß einer vorteilhaften Ausführungsform der vorliegenden Erfindung kann die Hubstange und die Zahnstange als Kolben-Zylinder-Einheit ausgestaltet sein. Hierdurch kann die Anbringung der Hubstange und der Zahnstange an der Halterung platzsparend umgesetzt werden.

Weitere Vorteile ergeben sich aus der folgenden Figurenbeschreibung. In den Figuren sind verschiedene Ausführungsbeispiele der vorliegenden Erfindung dargestellt. Die Figuren, die Beschreibung und die Ansprüche enthalten zahlreiche Merkmale in Kombination. Der Fachmann wird die Merkmale zweckmässigerweise auch einzeln betrachten und zu sinnvollen weiteren Kombinationen zusammenfassen.

In den Figuren sind gleiche und gleichartige Komponenten mit gleichen Bezugszeichen beziffert.

Es zeigen:
- Figur 1a: eine Vorderansicht auf einen menschlichen Rumpf mit einer Anzahl an schematisch dargestellten Gelenkpunkten;
- Figur 1b: eine weitere Vorderansicht auf einen menschlichen Rumpf mit einer Anzahl an schematisch dargestellten Gelenkpunkten;
- Figur 2: eine schematische Seitenansicht auf Bestandteile des erfindungsgemäßen Exoskeletts zum Stützen eines Arms eines Anwenders in einer ersten und zweiten Stellung;
- Figur 3: eine schematische Darstellung des erfindungsgemäßen Exoskeletts zum Stützen von zwei Armen des Anwenders;
- Figur 4: eine perspektivische Ansicht auf einen Ausleger, eine Halterung, ein Federelement und eine Hubstange des erfindungsgemäßen Exoskeletts in der ersten Einstellung;
- Figur 5: eine Seitenansicht auf den Ausleger, die Halterung, das Federelement und die Hubstange des erfindungsgemäßen Exoskeletts in der ersten Einstellung;
- Figur 6: eine perspektivische Ansicht auf den Ausleger, die Halterung, das Federelement und die Hubstange des erfindungsgemäßen Exoskeletts in der zweiten Einstellung;
- Figur 7: eine Seitenansicht auf den Ausleger, die Halterung, das Federelement und die Hubstange des erfindungsgemäßen Exoskeletts in der zweiten Einstellung;
- Figur 8: eine perspektivische Ansicht auf den Ausleger, die Halterung, das Federelement und die Hubstange des erfindungsgemäßen Exoskeletts in einer dritten Einstellung; und
- Figur 9: eine Seitenansicht auf den Ausleger, die Halterung, das Federelement und die Hubstange des erfindungsgemäßen Exoskeletts in der dritten Einstellung.

### Ausführunasbeispiel:

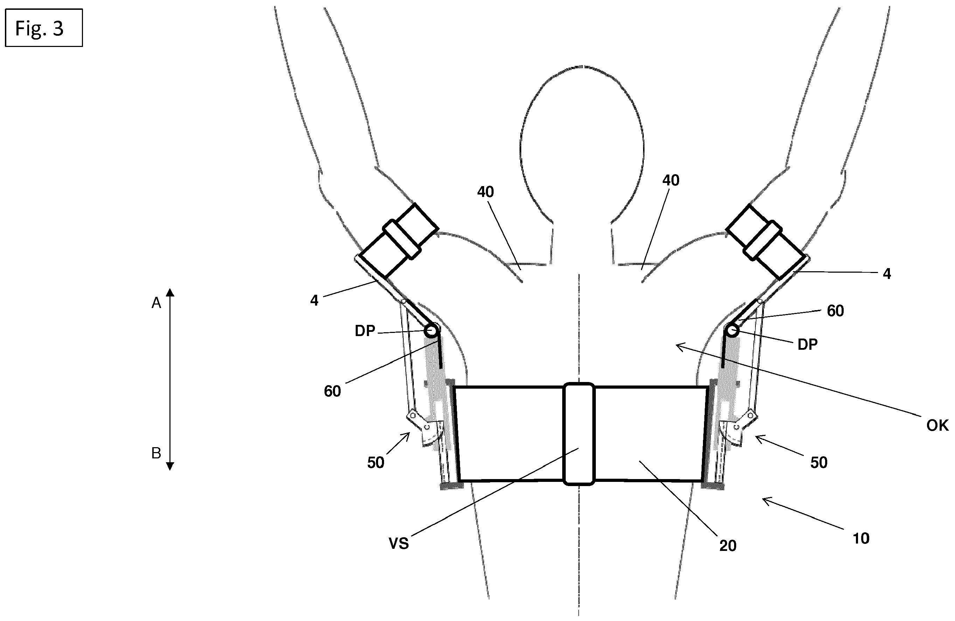

Figur 3 zeigt eine Vorderansicht auf einen menschlichen Oberkörper OK eines Anwenders AW, an dem ein Exoskelett 10 zur Unterstützung bzw. Steigerung der Arbeitsleistung der Arme 20 eines Anwenders AW angeordnet ist.

Das Exoskelett 10 enthält im Wesentlichen eine Rumpfanbindungsvorrichtung 30, eine Halterung 9, eine Hubstange 7, einen Ausleger 4 und ein Federelement 60.

Wie in den Figuren angedeutet kann zwischen dem Ausleger 4 und der Hubstange 7 ein Gelenkelement 1 vorgesehen sein, um den Ausleger 4 und die Hubstange 7 miteinander schwenkbar zu verbinden.

Der Rumpfanbindungsvorrichtung 30 dient zum wiederlösbaren Verbinden des Exoskeletts 10 mit einem Rumpf (d.h. dem Oberkörper OK) eines Anwenders AW (vgl. Figur 3). Die Rumpfanbindungsvorrichtung 30 kann dabei in Form einer Hartschale, eines flexiblen Brustgurtes bzw. Gürtels ausgestaltet sein. Des Weiteren enthält die Rumpfanbindungsvorrichtung 30 einen in der Mitte der Brust des Anwenders AW angeordneten Verschluss VS, mit dem die beispielsweise als Brustgurt ausgestaltete Rumpfanbindungsvorrichtung 30 geöffnet und verschlossen werden kann. Der Verschluss VS kann beispielsweise als Klettverschluss oder als Gürtelschnalle bzw. Gürtelschließe ausgestaltet sein.

Gemäß einer alternativen (in den Figuren nicht gezeigte) Ausgestaltungsform des erfindungsgemäßen Exoskeletts 10 kann die Rumpfanbindungsvorrichtung 30 auch in Form eines Hüftgürtels ausgestaltet sein und folglich an der Hüfte bzw. am Becken des Anwenders AW positioniert sein. Zusätzlich können ein bis zwei Gurte zur Fixierung der Rumpfanbindungsvorrichtung 30 an den Schultern 40 des Anwenders AW bzw. zum Verhindern eines Abrutschens der Rumpfanbindungsvorrichtung 30 in Pfeilrichtung B befestigt sein. Diese (in den Figuren nicht dargestellten) Gurte können von der Vorderseite der als Brustgurt bzw. Gürtel ausgestalteten Rumpfanbindungsvorrichtung 30 über die Schultern 40 des Anwenders AW (d.h. ähnlich wie Hosenträger) zu der Rückseite der Rumpfanbindungsvorrichtung 30 geführt sein.

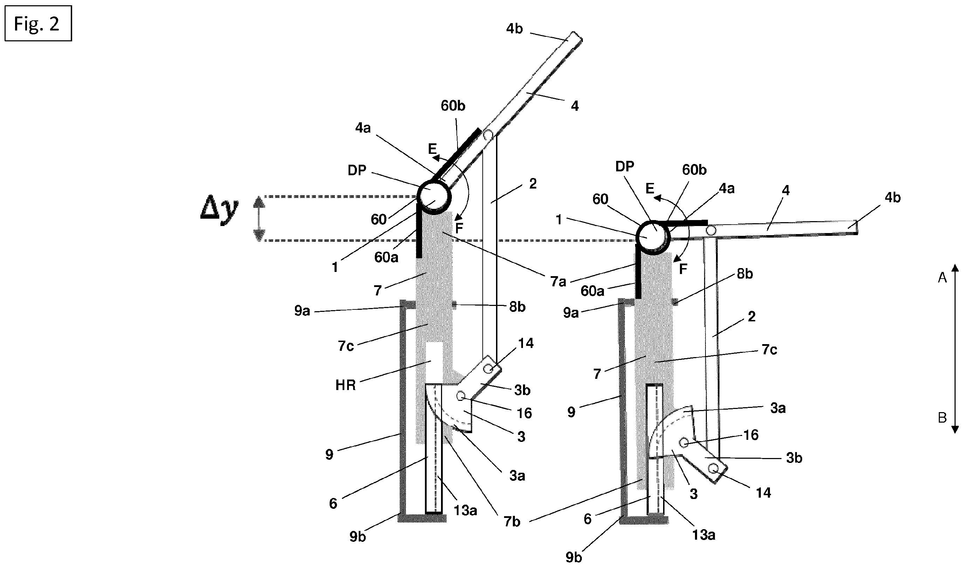

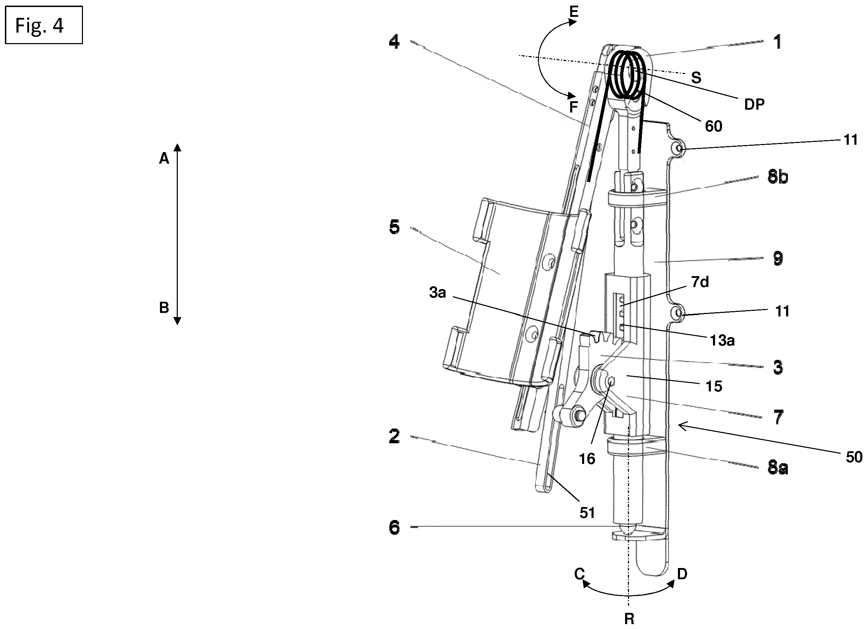

Wie in den Figuren 4 bis 9 gezeigt, ist die Halterung 9 im Wesentlichen als flaches Blech mit vier kreisrunden Aussparungen 11 sowie einem ersten Gleitlager 8a und zweiten Gleitlager 8b ausgestaltet. Die vier Aussparungen 11 können auch als Schraubdome bezeichnet werden und dienen zur seitlichen Anbindung der Halterung 9 an die Rumpfanbindungsvorrichtung 30. Hierfür können vier Schrauben oder Niete durch die vier Aussparungen 11 der Halterung 9 und zur Befestigung an der Rumpfanbindungsvorrichtung 30 getrieben werden. Darüber hinaus dient das erste und zweite Gleitlager 8a, 8b der Halterung 9 zur Aufnahme und Führung der Hubstange 7 an der Halterung 9. Die Schrauben und Niete sind in den Figuren nicht dargestellt. In Figur 2 ist hierbei lediglich das erste Gleitlager 8a gezeigt.

Jedes der Gleitlager 8a, 8b enthält eine kreisrunde Öffnung. Das erste Gleitlager 8a ist dabei so in der Nähe eines ersten Endes 9a der Halterung 9 und das zweit Gleitlager 8b ist so in der Nähe eines zweiten Endes 9b der Halterung 9 positioniert, dass die Hubstange 7 durch die jeweiligen kreisrunden Öffnungen der Gleitlager 8a, 8b geführt und hierdurch mit der Halterung 9 beweglich sowie reversibel in Pfeilrichtung A und B verbunden ist. Des Weiteren enthält die Halterung 9 an dem zweiten Ende 9b eine Stützplatte 12. Wie in Figur 4 gezeigt ist die Hubstange 7 auf der Stützplatte 12 drehbar gelagert, wenn die Hubstange 7 an der Halterung 9 positioniert ist. Alternativ kann die Stützplatte 12 auch eine zu der Hubstange 7 gerichtete konkave bzw. gebogene Form aufweisen.

Gemäß einer vorteilhaften Ausführungsform ist die Hubstange 7, wie in den Figuren 4 bis 8 gezeigt, im Wesentlichen als Röhre mit einem ersten Ende 7a und zweiten Ende 7b ausgestaltet. Diese Ausgestaltung der Hubstange 7 kann auch als Rohr oder Hülse bezeichnet werden. Wie insbesondere in Figur 4 ersichtlich erstreckt sich das erste Ende 7a der Hubstange 7 in Pfeilrichtung A über das erste Ende 9a der Halterung 9. In Figur 2 ist erkennbar, dass die als Röhre ausgestaltete Hubstange 7 einen Hohlraum HR enthält, welcher sich von dem zweiten Ende 7b bis ungefähr zur Mitte 7c der Hubstange 7 erstreckt. Der Hohlraum HR kann auch als Kavität, Sackloch oder Aussparung bezeichnet werden.

In den Hohlraum HR der Hubstange 7 kann gemäß einer vorteilhaften Ausführungsform ein Stützelement 6 eingeführt sein. Das Stützelement 6 kann zylindrisch ausgestaltet sein und in seiner Ausformung an den Hohlraum HR der Hubstange 7 angepasst sein. Die Hubstange 7 und das Stützelement 6 sind zueinander in Pfeilrichtung A und B reversibel beweglich. Das Stützelement 6 dient im Wesentlich zum Stützen der Hubstange 7 sowie zur Erhöhung der Stabilität, wenn die Hubstange 7 relativ zur Halterung 9 bewegt wird. Wie später noch beschrieben, kann das Stützelement 6 auch mit Zähnen und damit als Zahnstange ausgestaltet sein.

Wie bereits vorstehend erwähnt ist die Hubstange 7 mit der Halterung 9 so verbunden, dass sich die Hubstange 7 durch die Öffnungen des ersten und zweiten Gleitlagers 8a, 8b erstreckt. Die Hubstange 7 ist hierdurch in eine erste Pfeilrichtung A und eine zweite Pfeilrichtung B reversibel und relativ zu der Halterung 9 beweglich. Mit anderen Worten: die Hubstange 7 kann in Pfeilrichtung A und in Pfeilrichtung B relativ zu der Halterung 9 bewegt werden. Außerdem kann die Hubstange 7 mittels dem ersten und zweiten Gleitlager 8a, 8b relativ zu der Halterung 9 um die Drehachse R in Drehrichtung C oder D gedreht werden.

Der Ausleger 4 ist im Wesentlichen als Stange mit einem ersten Ende 4a und zweiten Ende 4b ausgestaltet. Die Stange kann auch als Arm, Stütze, Stab oder Hebel bezeichnet werden. Wie in den Figuren 4 bis 9 dargestellt ist eine Armauflage 5 an dem Ausleger 4 positioniert. Die Armauflage 5 dient zum Aufnehmen und Halten eines Oberarms eines Anwenders AW. Um den Arm an der Armauflage 5 zeitweilig zu fixieren, ist ein Armgurt mit einem Verschluss vorgesehen. Der Armgurt und der Verschluss sind in Figur 3 dargestellt. Der Verschluss ist dabei beispielsweise als Klettverschluss ausgestaltet.

Wie in den Figuren 2 bis 9 gezeigt ist das Federelement 60 als Schenkelfeder mit einem ersten Schenkel 60a und einem zweiten Schenkel 60b ausgestaltet. Das Federelement 60 ist dabei so zwischen dem Ausleger 4 und der Hubstange 7 positioniert, dass der erste Schenkel 60a an der Hubstange 7 und der zweite Schenkel 60b an dem Ausleger 4 anliegt. Die Federkraft des als Schenkelfeder ausgestalteten Federelements 60 bewirkt damit, dass der Ausleger 4 und die Hubstange 7 in Drehrichtung E voneinander weggedrückt werden. Wie in den Figuren angedeutet ist das Federelement 60 um das Gelenkelement 1 positioniert.

Dadurch, dass die Federkraft des Federelements 60 kontinuierlich den Ausleger 4 in Drehrichtung E und damit von der Hubstange 7 wegdrücken will, unterstützt bzw. drückt das Federelement 60 einen Arm eines Anwenders AW, der über den Armaufleger 5 mit dem Ausleger 4 verbunden ist, in Drehrichtung E. Hierdurch werden die Arme unterstützt und eine Last, beispielsweise eine in den Armen gehaltene Werkzeugmaschine, nach oben in Pfeilrichtung A gedrückt. Die Last erscheint hierdurch leichter und der Anwender AW kann die Last länger halten. Die Gewichtskraft der Last (z.B. Werkzeugmaschine) wird hierdurch über die Arme auf den Ausleger 4, die Hubstange 7, die Halterung 9 und schließlich auf die Rumpfanbindungsvorrichtung 30 abgeleitet. Die Werkzeugmaschine ist in den Figuren nicht dargestellt.

Gemäß einer weiteren vorteilhaften Ausgestaltung enthält das Exoskelett 10 des Weiteren eine Koppelstange 2, welche im Wesentlichen als Stab mit einem ersten Ende 2a und zweiten Ende 2b ausgestaltet ist (vgl. Figur 4 bis 9). Die Koppelstange 2 ist mit dem ersten Ende 2a drehbar an dem Ausleger 4 und wiederum drehbar mit dem zweiten Ende 2b über einen Ausgleichsmechanismus 50 mit der Hubstange 7 verbunden ist. Die Koppelstange 2 dient im Wesentlichen als Stütze und Führung, wenn sich der Ausleger 4 relativ zu der Hubstange 7 bewegt.

Gemäß einer vorteilhaften (in den Figuren nicht gezeigten) Ausgestaltungsform des erfindungsgemäßen Exoskeletts 10 kann der Ausgleichsmechanismus 50 in Form eines Langlochs 51 am zweiten Ende der Koppelstange 2 sowie eines Drehlagers an der Hubstange 7 ausgestaltet sein. Das Drehlager ist dabei in dem Langloch 51 positioniert und entlang dem Langloch 51 bzw. durch das Langloch 51 geführt beweglich. Mit Hilfe des als Langloch und Drehlager ausgestalteten Ausgleichsmechanismus 50 kann eine Bewegung der Koppelstange 2, wenn der Ausleger 4 relativ zu der Hubstange 7 gedreht wird, ausgeglichen werden.

Gemäß einer weiteren und in den Figuren 4 bis 9 gezeigten Ausgestaltungsform des erfindungsgemäßen Exoskeletts 10 kann der Ausgleichsmechanismus 50 in Form eines Langlochs 51 am zweiten Ende 2b der Koppelstange 2, einem Sektorgetriebe 3, einer Zahnstange 13 sowie eines Drehlagers 14 ausgestaltet sein.

Das optionale Stützelement 6 ist gemäß der in Figur 4 bis 9 gezeigten Ausgestaltungsform des erfindungsgemäßen Exoskeletts 10 in Form der Zahnstange 13 ausgestaltet. Es ist gemäß einer vorteilhaften Ausgestaltungsform des erfindungsgemäßen Exoskeletts 10 auch möglich, dass sowohl ein Stützelement 6 als auch eine Zahnstange 13 nebeneinander auf der Stützplatte 12 positioniert sind.

Die zylindrisch ausgestaltete Zahnstange 13 ragt in das Inneren der Hubstange 7 so hinein, dass die Längserstreckung der Zahnstange 13 und die Längserstreckung der Hubstange 7 parallel zueinander ausgerichtet sind.

Wie insbesondere in Figur 4 ersichtlich weist die Hubstange 7 eine Aussparung 7d auf, durch die ein Zugang zu den Zähnen der Zahnstange 13 gegeben ist. Die Zahnstange 13 ist in Drehrichtung C oder D mittels eines (nicht gezeigten) Drehlagers drehbar um die Drehachse R mit dem zweiten Ende 7b der Hubstange 7 und insbesondere mit der Stützplatte 12 am zweiten Ende 7b der Hubstange 7 verbunden. Die Zahnstange 13 ist dabei so mit der Stützplatte 12 verbunden, dass die Zahnstange 13 nicht in Pfeilrichtung A oder B bewegt werden kann Halterung. Es kommt hierdurch nicht zu einer Relativbewegung zwischen der Zahnstange 13 und der Halterung 9.

Durch die Möglichkeit, dass sich die Zahnstange 13 und die Hubstange 7 relativ zueinander in Pfeilrichtung A oder B bewegen können sowie dadurch, dass die Zahnstange 13 im Inneren der Hubstange 7 eingeführt ist, kann die Kombination bzw. das Zusammenwirken von der Zahnstange 13 und der Hubstange 7 als Kolben-Zylinder-Einheit bezeichnet werden. Hierbei ist die Zahnstange 13 als Kolben und die Hubstange 7, und insbesondere der Hohlraum HR der Hubstange 7, als Zylinder zur Aufnahme des als Zahnstange ausgestalteten Kolbens ungesetzt sind.

Des Weiteren enthält die Hubstange 7 eine Haltevorrichtung 15 mit einer Achse 16, welche zum Aufnehmen und Halten des Sektorgetriebes 3 dient. Durch die Haltevorrichtung 15 mit der Achse 16 kann das Sektorgetriebe 3 relativ zu der Hubstange 7 so gedreht werden, dass die Zähne 3a des Sektorgetriebes 3 in die Zähne 13 der Zahnstange 13 greifen können. Das Sektorgetriebe 3 enthält darüber hinaus ein Hebelelement 3b an dessen freien Ende das Drehlager 14 positioniert ist. Das Drehlager 14 ist in dem Langloch 51 positioniert und entlang dem Langloch 51 frei beweglich bzw. entlang dem Langloch 51 geführt.

Der Ausleger 4 kann durch seine Drehlagerung am Drehpunkt DP zur Halterung 9 reversibel in eine erste, zweite oder dritte Stellung gebracht werden.

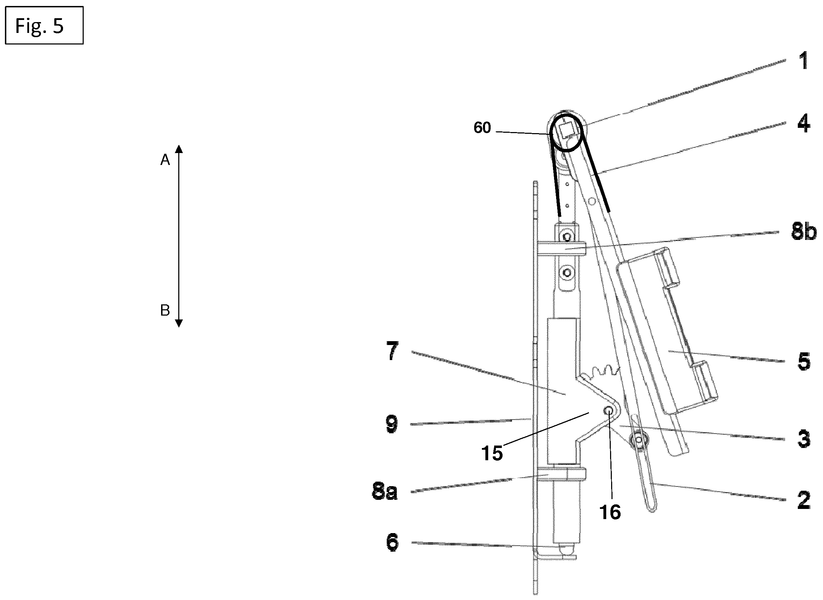

In Figur 4 und 5 ist das Exoskelett 10 mit dem Ausleger 4 in der ersten Stellung gezeigt.

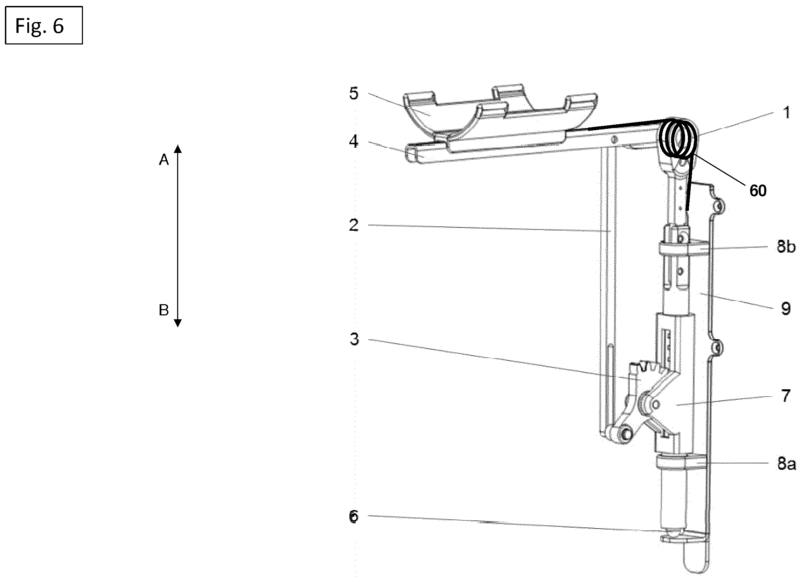

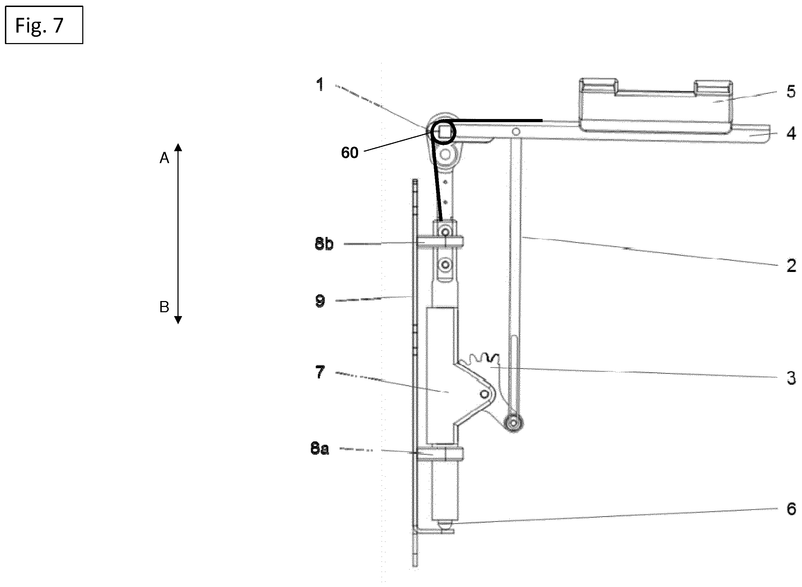

Figur 6 und 7 zeigen den Ausleger 4 in der zweiten Stellung.

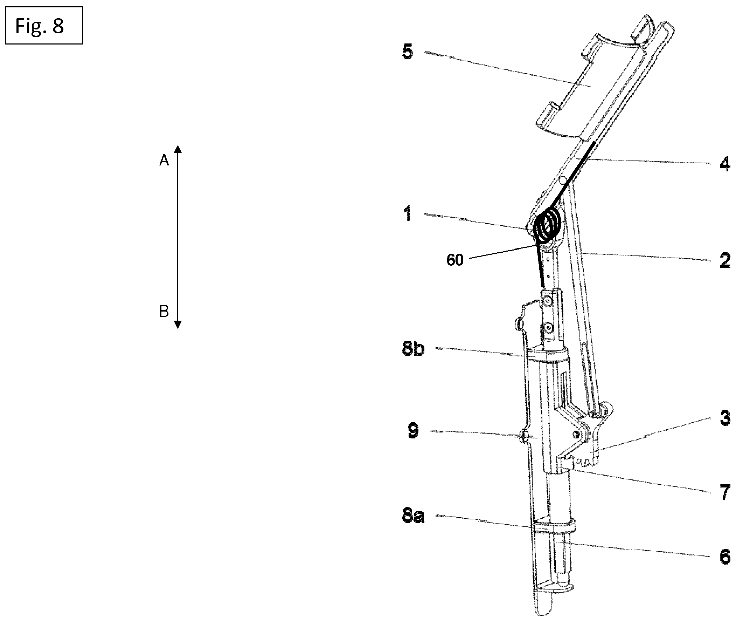

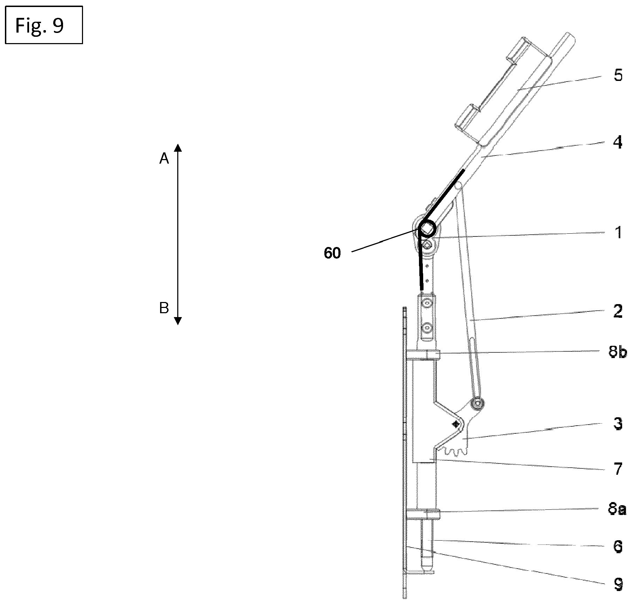

In den Figuren 8 und 9 ist der Ausleger 4 in der dritten Stellung dargestellt.

In der ersten Stellung ist das zweite Ende 4b des Auslegers 4 nach unten in Pfeilrichtung B geneigt.

In der zweiten Stellung steht das zweite Ende 4b des Auslegers 4 im Wesentlichen waagerecht. In der dritten Stellung zeigt das erste Ende 4a des Auslegers 4 nach oben in Pfeilrichtung A.

Wenn der Anwender AW den Arm, welcher mit dem Ausleger 4 verbunden ist, nach oben in Pfeilrichtung A bewegt, wird über das Gelenkelement 1 die Hubstange 7 ebenfalls nach oben in Pfeilrichtung A gezogen. Dadurch, dass die Hubstange 7 zu der Halterung 9 in Pfeilrichtung A oder B beweglich gelagert ist, kann der Drehpunkt DP die Wegstrecke Δy zum Ausgleich der anatomischen Bewegung der menschlichen Schulter 40 beim Anheben eines Armes ausgleichen. Ein derartiger Ausgleich passt das Exoskelett an die tatsächliche Anatomie bzw. den tatsächlichen Bewegungsablauf des menschlichen Anwenders AW beim Heben von Lasten besser an und erhöht den Tragekomfort des Exoskeletts.

### Bezugszeichenliste

- 1:: Gelenkelement
- 2:: Koppelstange
- 2a:: erstes Ende der Koppelstange
- 2b:: zweites Ende der Koppelstange
- 3:: Sektorgetriebe
- 3a:: Zähne des Sektorgetriebes
- 3b:: Hebelelement
- 4:: Ausleger
- 4a:: erstes Ende des Auslegers
- 4b:: zweites Ende des Auslegers
- 5:: Armauflage
- 6:: Stützelement
- 7:: Hubstange
- 7a:: erstes Ende der Hubstange
- 7b:: zweites Ende der Hubstange
- 7c:: Mitte der Hubstange
- 7d:: Aussparung an der Hubstange
- 8a:: erstes Gleitlager
- 8b:: zweites Gleitlager
- 9:: Halterung
- 9a:: erstes Ende der Halterung
- 9b:: zweites Ender Halterung
- 10:: Exoskelett
- 11:: Aussparungen an Halterung
- 12:: Stützplatte
- 13:: Zahnstange
- 13a:: Zähne der Zahnstange
- 14:: Drehlager
- 15:: Haltevorrichtung
- 16:: Achse der Haltevorrichtung
- 20:: Arm des Anwenders
- 30:: Rumpfanbindungsvorrichtung
- 40:: Schultern des Anwenders
- 50:: Ausgleichsmechanismus
- 51:: Langloch
- 60:: Federelement
- 60a:: erster Schenkel des Federelements
- 60b:: zweiter Schenkel des Federelements
- HR:: Hohlraum der Hubstange
- DP:: Drehpunkt
- OK:: Oberkörper
- AW:: Anwender
- VS:: Verschluss
- R:: Drehachse der Hubstange
- C, D:: Drehrichtungen der Hubstange
- S:: Drehachse des Auslegers
- E, F:: Drehrichtungen des Auslegers

## Patentansprüche

1. Exoskelett (10) zum Stützen wenigstens eines Arms (20) eines Anwenders (AW),
**gekennzeichnet durch**
- eine Rumpfanbindungsvorrichtung (30) zum wiederlösbaren Verbinden des Exoskeletts (10) mit einem Rumpf eines Anwenders (AW);
- wenigstens eine Halterung (9), welche mit der Rumpfanbindungsvorrichtung (30) verbindbar ist;
- eine Hubstange (7), welche mittels der wenigstens eine Halterung (9) mit der Rumpfanbindungsvorrichtung (30) verbindbar ist und welche reversibel in eine erste und zweite Richtung (A, B) relativ zu der Halterung (9) bewegbar ist;
- einen Ausleger (4) zum Stützen des Arms (20) eines Anwenders (AW), welcher mit dem Arm (20) eines Anwenders (AW) wiederlösbar verbindbar ist; und
- ein Federelement (60) zum Ausüben einer Kraft auf den Ausleger (4) und die Hubstange (7).

2. Exoskelett (10) nach Anspruch 1,
**gekennzeichnet durch** eine Koppelstange (2) mit einem ersten und zweiten Ende (2a, 2b), welche mit dem ersten Ende (2a) an dem Ausleger (4) und dem zweiten Ende (2b) über einen Ausgleichsmechanismus (50) mit der Hubstange (7) jeweils drehbar verbunden ist.

3. Exoskelett (10) nach Anspruch 2,
**dadurch gekennzeichnet, dass** der Ausgleichsmechanismus (50) in Form eines Langlochs (51) am zweiten Ende (2b) der Koppelstange (2) und eines Drehlagers (14) an der Hubstange (7) ausgestaltet ist, wobei das Drehlager (14) in dem Langloch (51) positioniert ist.

4. Exoskelett (10) nach Anspruch 2,
**dadurch gekennzeichnet, dass** der Ausgleichsmechanismus (50) in Form Kulissensteuerung zwischen dem zweiten Ende (2b) der Koppelstange (2) und der Hubstange (7) ausgestaltet ist.

5. Exoskelett (10) nach Anspruch 2,
**dadurch gekennzeichnet, dass** der Ausgleichsmechanismus (50) in Form in eines Sektorgetriebes (3) sowie einer Zahnstange (13) ausgestaltet ist, wobei das Sektorgetriebe (3) drehbar mit der Hubstange (7) verbunden ist, sodass die Zähne (3a) des Sektorgetriebes (3) in die Zähne (13a) der Zahnstange (13) eingreifen.

6. Exoskelett (10) nach Anspruch 4,
**dadurch gekennzeichnet, dass** die Hubstange (7) und die Zahnstange (13) als Kolben-Zylinder-Einheit ausgestaltet ist.
